# EUROPEAN PATENT APPLICATION

(11) **EP 4 477 212 A1**
(43) Date of publication of application: **18.12.2024**
(21) Application number: 23382600.7
(22) Date of filing: 16.06.2023
(51) Int. Cl.: A61K 8/41, A61K 8/49, A61Q 17/04, A61Q 19/02, A61Q 19/08

(54) **BLUE-LIGHT ABSORBING COMPOUNDS AND COMPOSITIONS**

(71) Applicant: Esencias Moles, S.A., 08758 Cervelló, Barcelona (ES)
(72) Inventor: RUIZ GONZÁLEZ, Rubén, 08758 Cervelló, Barcelona (ES)
(74) Representative: ABG Intellectual Property Law, S.L.

(57) **Abstract**

The present invention relates to the cosmetic use of compounds of formula (I) and cosmetic compositions comprising said compounds in the treatment and/or prevention of signs of skin aging, in particular signs of skin photoaging caused by blue light. The present invention also relates to methods for the cosmetic treatment and/or prevention of signs of skin aging, in particular signs of skin photoaging caused by blue light, comprising the topical application of compounds of formula (I) and cosmetic compositions comprising said compounds.

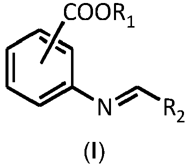

## Description

### FIELD OF THE INVENTION

The present invention relates to the cosmetic use of blue-light absorbing compounds and cosmetic compositions comprising said compounds in the treatment and/or prevention of signs of skin aging, in particular signs of skin photoaging caused by blue light. The present invention also relates to methods for the cosmetic treatment and/or prevention of signs of skin aging, in particular signs of skin photoaging caused by blue light, comprising the topical application of blue-light absorbing compounds and cosmetic compositions comprising said compounds.

### BACKGROUND OF THE INVENTION

There is a growing interest in the skin care and personal care world to address problems in the antipollution segment. Among them, long-term exposure to blue light is known to be responsible for premature skin aging.

Blue light is emitted visible light between the wavelengths of 400 to 500 nm. Blue light is generally referred to as high energy visible light as it is at the shortest wavelength, thus the highest energy, in the visible light spectrum. The main source of blue light is sunlight, but digital screens, such as cellphones, computers, laptops and TVs, light-emitting diodes (LEDs), and fluorescent lighting serve as additional sources.

Whether from sunlight or from artificial blue light of electronic devices, blue light is all around us all the time and blue light exposure is inevitable. Daily time spent in front of electronic devices (i.e. artificial blue light) is increasing to the point where many countries are exposed for four hours per day on average. This exposes users to blue light that affects sleep and reduces skin's antioxidant levels.

Blue light irradiation of human skin has been reported to cause skin aging [Coats J.G. et al., J. Cosmet. Dermatol., 2021, 20, 714-717; Panda P. et al., Research Journal Pharmacy Life Sciences, 2021, 2(2), 43-58; Suitthimeathegorn O. et al., Skin Pharmacol. Physiol., 2022, 35, 305-318; Kumari J. et al., J. Cosmet. Dermatol., 2022, 00, 1-6]. In particular, it has been reported to result in the formation of free radicals due to the decrease in carotenoids. This oxidative stress on melanogenic precursors has been reported to lead to pigmentation changes, such as immediate and persistent darkening. Studies have also shown that blue light exposure can stimulate melanocytes and lead to pigmentation issues, such as melasma and age spots. Blue light exposures can also lead to the formation of reactive oxygen species (ROS) which contribute to skin aging, hyperpigmentation and melasma. Blue light has also a negative effect on collagen and elastin, leading to skin laxity, sagging, early aging and wrinkles.

Several approaches to prevent blue-light related damage have already been proposed, for example algae-derived ingredients, botanical extracts, antioxidants, vitamins and blue light absorbers [Coats J.G. et al., J. Cosmet. Dermatol., 2021, 20, 718-723*;* Kumari J. et al., J. Cosmet. Dermatol., 2022, 00, 1-6]. Carotolino is an oil soluble active ingredient, preservative-free/ self-preserving based on the combination of carrot root extract, carrot seed oil and β-carotene, all united in a basis of canola oil in order to provide stabilized carotenoids. Carotolino shows efficacy in shielding blue light, neutralizing blue light-induced reactive oxygen species. In this line, Rubescens Algae extract (PephaAge) is another example of carotenoid rich composition. Dragosine^{®} (L-Carnosine) is a nature-identical dipeptide (β-Alanyl-L-histidin). In cosmetic products, L-Carnosine is used as an anti-aging ingredient. It has been shown to have excellent results in the prevention of hyperpigmentation and oxidative damage induced by high-energy visible light. However, its mechanism does not seem to be due to blue-light filtering but rather on reactive oxygen species quenching. Among the synthetic options, AC_GR (acetyl glucosyl rutin) is a water-soluble approach meant to absorb in this light interval. Also, different inorganic materials such as titanium, zinc or cerium oxides have been shown to exhibit capacity to filter light in the blue region. However, their difficulty in incorporation in formulations is quite limited and complex.

Nevertheless, there is still the need to provide further compounds and cosmetic compositions capable of filtering blue-light and, therefore, of use in a wide range of cosmetic products to prevent and treat signs of skin aging.

### SUMMARY OF THE INVENTION

The inventors have surprisingly found a family of compounds showing effective absorption in the 400-500 nm wavelength interval. Thus, these blue light absorbing compounds are useful for cosmetic treatment and/or prevention of skin aging. Moreover, as shown in the examples, these compounds are stable in the formulations including them, in particular with respect to photostability.

In the first aspect, the present invention relates to a cosmetic (non-therapeutic) use of a compound of formula (I) 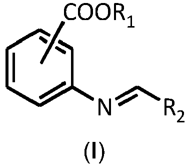 wherein
R₁ is selected from the group consisting of linear or branched C₁-C₆ alkyl, linear or branched C₂-C₆ alkenyl and hydrogen atom;
R₂ is selected from the group consisting of:
   - linear or branched C₁-C₁₂ alkyl optionally substituted by one or more substituents selected from the group consisting of -OH; phenyl which is in turn optionally substituted by one or more substituents selected from -OH, -OR₃ and R₃; benzodioxolyl;
   - linear or branched C₂-C₁₂ alkenyl optionally substituted by one or more substituents selected from the group consisting of -OH and phenyl;
   - C₅-C₆ cycloalkenyl optionally substituted by one or more substituents selected from the group consisting of linear or branched C₁-C₆ alkyl and linear or branched C₂-C₆ alkenyl and wherein said alkyl and alkenyl are in turn optionally substituted by a -OH;
   - C₅-C₆ cycloakyl optionally substituted by one or more substituents selected from the group consisting of linear or branched C₁-C₆ alkyl and linear or branched C₂-C₆ alkenyl and wherein said alkyl and alkenyl are in turn optionally substituted by a -OH;
   - phenyl optionally substituted by -OH and -OR₆;
R₃ is independently a linear or branched C₁-C₆ alkyl; and
R₆ is a linear or branched C₁-C₃ alkyl;
or a cosmetically acceptable salt, stereoisomer or metal complex thereof, in the treatment and/or prevention of signs of skin aging, in particular signs of skin photoaging caused by blue light.

In the second aspect, the invention relates to a cosmetic use of a cosmetic composition comprising a compound of formula (I) or a cosmetically acceptable salt, stereoisomer or metal complex thereof as defined in the first aspect and a cosmetically acceptable excipient in the treatment and/or prevention of signs of skin aging, in particular signs of photoaging caused by blue light.

In the third aspect, the invention relates to a method for the cosmetic (non-therapeutic) treatment and/or prevention of signs of skin aging, in particular signs of skin photoaging caused by blue light, comprising the topical application of a compound of formula (I) or a cosmetically acceptable salt, stereoisomer or metal complex thereof as defined in the first aspect, or of a cosmetic composition as defined in the second aspect.

### DESCRIPTION OF THE FIGURES

Figure 1 shows the photostability of Schiff bases by comparing two different Schiff Base ethanolic solutions which have been exposed to light. From left to right, i) Phenyl acetaldehyde / methyl anthranilate Schiff base; ii) Hydroxycitronellal / methyl anthranilate Schiff base.
Figure 2 shows (on top) the normalized spectra of phenylacetaldehyde, methyl anthranilate and its Schiff Base (SB 3); and (at the bottom) the normalized spectra of C11 aldehyde (i.e. 10-undecenal), methyl anthranilate and its Schiff Base (SB 9).
Figure 3 shows the absorption spectra in ethanol of the different Schiff Bases described in the invention SB 1 to SB 5 (top) and SB 6 to SB 10 (bottom).
Figure 4 shows the absorption spectra of fragranced hydroalcoholic lotions without (solid line) or with increased concentrations of Schiff base SB3 (open lines).

### DETAILED DESCRIPTION OF THE INVENTION

As explained above, the inventors have found a family of compounds showing effective absorption in the 400-500 nm wavelength interval. Thus, these blue light absorbing compounds are useful for the cosmetic treatment and/or prevention of skin aging.

Thus, in the first aspect, the invention relates to a cosmetic use of a compound of formula (I) 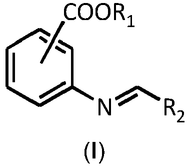 wherein
R₁ is selected from the group consisting of linear or branched C₁-C₆ alkyl, linear or branched C₂-C₆ alkenyl, and hydrogen atom;
R₂ is selected from the group consisting of:
   - linear or branched C₁-C₁₂ alkyl optionally substituted by one or more substituents selected from the group consisting of -OH; phenyl which is in turn optionally substituted by one or more substituents selected from -OH, -OR₃ and R₃; benzodioxolyl;
   - linear or branched C₂-C₁₂ alkenyl optionally substituted by one or more substituents selected from the group consisting of -OH and phenyl;
   - C₅-C₆ cycloalkenyl optionally substituted by one or more substituents selected from the group consisting of linear or branched C₁-C₆ alkyl and linear or branched C₂-C₆ alkenyl and wherein said alkyl and alkenyl are in turn optionally substituted by a -OH;
   - C₅-C₆ cycloakyl optionally substituted by one or more substituents selected from the group consisting of linear or branched C₁-C₆ alkyl and linear or branched C₂-C₆ alkenyl and wherein said alkyl and alkenyl are in turn optionally substituted by a -OH;
   - phenyl optionally substituted by -OH and -OR₆;
R₃ is independently a linear or branched C₁-C₆ alkyl; and
R₆ is a linear or branched C₁-C₃ alkyl;
or a cosmetically acceptable salt, stereoisomer or metal complex thereof, in the treatment and/or prevention of signs of skin aging.

In the context of the present invention, the expression "cosmetic use" refers to a non-therapeutic use.

In the context of the present invention, the term "treatment" refers to a non-therapeutic cosmetic treatment, in which the application of the compounds and compositions of the invention on improves the cosmetic appearance of the skin with respect to signs of aging, in particular with respect to signs of skin photoaging caused by blue light, such as by improving the number and/or depth of wrinkles, the level of hyperpigmentation, the number and/or area of age spots and the level of sagging of the skin.

In the context of the present invention, the term "prevention" refers to the capability of the compounds and compositions of the invention to prevent, delay, or impede the onset of skin aging, in particular skin photoaging caused by blue light, preferably with respect to wrinkles, hyperpigmentation, age spots and sagging of the skin. The compounds and compositions of the present invention are preferably used for the prevention of signs of skin aging, in particular of signs of skin photoaging caused by blue light.

Preferably, the signs of skin aging refer to wrinkles, hyperpigmentation, age spots and sagging of the skin.

Preferably, the compound of formula (I) is topically applied to the skin, preferably one, two or three times a day.

The term "wrinkles" refers to lines and creases that form in the skin.

The term "hyperpigmentation" refers to areas of the skin that are darker than the surrounding skin. Hyperpigmentation may appear as freckles, age spots or larger areas of darkened skin.

The term "age spots" refers to spots on the skin ranging from light brown to black in color. They have the same texture as the rest of the skin and are flat to the touch. They usually occur on the face and on the backs of the hands. They are most common in people over the age of 40 but they can occur earlier.

The term "sagging of the skin" refers to loose skin that has little to no definition due to a reduction in collagen and elastin proteins.

In a particular embodiment, the cosmetic use is applied to a human subject, in particular to a human subject who has not been exposed to any level of UV and visible radiation harmful to the health of said subject.

"Blue light" is emitted visible light between the wavelengths of 400 to 500 nm.

The term "alkyl" as used herein alone or as part of another group designates both linear- and branched-chain saturated hydrocarbons containing the number of carbon atoms indicated along the invention and attached to the rest of the molecule through a single bond. Examples of alkyl groups are methyl, ethyl, n-propyl, isopropyl, n-butyl, isobutyl, tert-butyl, pentyl, hexyl, isohexyl, heptyl, 4,4-dimethylpentyl, octyl, 2,2,4-trimethylpentyl, nonyl, decyl, among others. The alkyl groups can be optionally substituted by one or more substituents, preferably one or two, wherein said substituents are as indicated along the invention.

The term "alkenyl" as used herein alone or as part of another group designates both linear- and branched-chain hydrocarbons containing the number of carbon atoms indicated along the invention, having one or more double bonds (such as one, two, three, four, five or six, preferably one, two or three), and attached to the rest of the molecule through a single bond. Examples of alkenyl groups are 2,6-dimethylhept-1,5-dien-1-yl, 9-decen-1-yl. The alkenyl groups can be optionally substituted by one or more substituents, preferably one or two, wherein said substituents are as indicated along the invention.

The term "cycloalkyl" as used herein alone or as part of another group refers to saturated monocyclic hydrocarbon group, and containing from 5 to 6 carbon atoms forming part of the ring system. Examples of cycloalkyl groups are cyclohexyl and cyclopentyl, preferably cyclohexyl. The cycloalkyl groups can be optionally substituted by one or more substituents, preferably one or two, wherein said substituents as indicated along the invention.

The term "cycloalkenyl" as used herein alone or as part of another group refers to monocyclic hydrocarbon group comprising one or two double bonds, preferably one, and containing from 5 to 6 carbon atoms forming part of the ring system. Examples of cycloalkenyl groups are cyclohexenyl and cyclopentenyl, preferably cyclohexenyl. The cycloalkenyl groups can be optionally substituted by one or more substituents, preferably one or two, wherein said substituents are as indicated along the invention.

The expression "one or more substituents" as used herein preferably refers to 1, 2, 3, 4, 5, or 6 substituents, more preferably 1 or 2 substituents.

The term cosmetically acceptable salt as used herein refers to salts with a cosmetically acceptable acid or base, which are synthesized from the parent compound which contains an acidic moiety by addition of a cosmetically acceptable base, or which are synthesized from the parent compound which contains a basic moiety by addition of a cosmetically acceptable acid. Cosmetically acceptable acids include both inorganic acids, for example, hydrochloric (HCl), sulfuric (H₂SO₄), phosphoric (H₃PO₄), diphosphoric (H₄P₂O₇), hydrobromic (HBr), hydroiodic (HI), and nitric acid (HNO₃), and organic acids, for example, citric, lactic, fumaric, maleic, malic, mandelic, ascorbic, oxalic, succinic, tartaric, benzoic, acetic (AcOH), methanesulfonic, ethanesulfonic, benzenesulfonic, or p-toluenesulfonic acid. Cosmetically acceptable bases include alkali metal (e.g., sodium or potassium) and alkali earth metal (e.g., calcium or magnesium) hydroxides and organic bases, such as alkyl amines, arylalkyl amines, and heterocyclic amines.

All stereoisomers of the compounds defined herein are contemplated either alone or as mixtures thereof. Stereoisomers refer to compounds having stereogenic centres, e.g. enantiomers, diastereomers, meso and racemic forms, and to compounds having different substituents on the atoms linked to form a multiple bond, such as a double bond (e.g. -HC=CH-), i.e. cis (Z) and trans (E) isomers. When diastereomeric or enantiomeric products are prepared, they can be separated by conventional methods, for example, chromatographic or functional crystallization.

Since the nitrogen atom in the imine bond of the compounds of formula (I) contains unpaired electrons, these compounds can form complexes with almost all transition metals. Also, the carboxylic group in the compounds of formula (I) may act as a ligand for these metals. In particular, the metal complexes are formed with a metal selected from the group consisting of Zn(II), Bi(III), Ag(I), Ag(II), Fe(II), Fe(III), Co(II),Cu(II), Mn(II), Al (III), Ni(II), Cr(III), Pb(II), Gd (III), Y(II) and Hg(II). The metal complexes can be prepared from the parent compound by treatment with a salt of the corresponding metal.

Preferably, in the compound of formula (I), the -COOR₁ group is in ortho position with respect to the -N=CH-R₂ moiety, i.e. the compound has formula (Ia) depicted below:

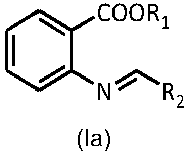

Preferably, in the compounds of formula (I), R₁ is selected from the group consisting of linear or branched C₁-C₆ alkyl and linear or branched C₂-C₆ alkenyl. Preferably R₁ is C₁-C₃ alkyl, such as methyl, ethyl, n-propyl or isopropyl, more preferably methyl.

Preferably, in the compounds of formula (I), R₂ is selected from the group consisting of:
- linear or branched C₇-C₁₁ alkyl optionally substituted by one -OH;
- linear or branched C₇-C₁₁ alkenyl optionally substituted by one phenyl;
- cyclohexenyl optionally substituted by one or two substituents selected from the group consisting of linear or branched C₁-C₆ alkyl and linear or branched C₂-C₆ alkenyl;
- cyclohexyl optionally substituted by one substituent selected from the group consisting of linear or branched C₁-C₆ alkyl which is in turn optionally substituted by a -OH;
- phenyl optionally substituted by methoxy;
- benzyl;
- phenylethenyl; and
- a substituent of formula (II): 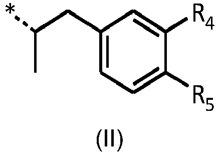 wherein
   the dotted line ending in * represents the attachment point to the rest of the molecule; and
   either R₄ is H and R₅ is selected from the group consisting of -OR₃, R₃ and OH, wherein R₃ is independently a linear or branched C₁-C₆ alkyl;
   or R₄ and R₅ form together a -O-CH₂-O- group.

In a particular embodiment, in the compounds of formula (I), R₂ is selected from the group consisting of:
- linear or branched C₇-C₁₁ alkyl optionally substituted by one -OH;
- cyclohexyl optionally substituted by one substituent selected from the group consisting of linear or branched C₁-C₆ alkyl which is in turn optionally substituted by a -OH;
- benzyl; and
- a substituent of formula (II): 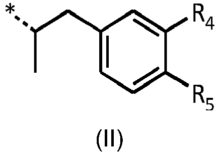 wherein
   the dotted line ending in * represents the attachment point to the rest of the molecule; and
   either R₄ is H and R₅ is selected from the group consisting of -OR₃, R₃ and OH, wherein R₃ is independently a linear or branched C₁-C₆ alkyl;
   or R₄ and R₅ form together a -O-CH₂-O- group.

In another particular embodiment, in the compounds of formula (I), R₂ is selected from the group consisting of:
- linear or branched C₇-C₁₁ alkyl optionally substituted by one -OH; and
   - cyclohexyl optionally substituted by one substituent selected from the group consisting of linear or branched C₁-C₆ alkyl which is in turn optionally substituted by a -OH.

Preferably, in the compounds of formula (I), R₂ is selected from the group consisting of:
- linear or branched C₉-C₁₁ alkyl optionally substituted by one -OH;
- linear or branched C₈-C₁₀ alkenyl optionally substituted by one phenyl;
- cyclohexenyl optionally substituted by one or two substituents selected from the group consisting of linear or branched C₁-C₃ alkyl;
- phenyl optionally substituted by methoxy; and
- substituent of formula (II) 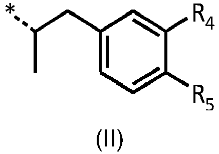
wherein either R₄ is H and R₅ is selected from the group consisting of methoxy and linear or branched C₃-C₆ alkyl; or R₄ and R₅ form together a -O-CH₂-O-group.

In a particular embodiment, in the compounds of formula (I), R₂ is selected from the group consisting of:
- linear or branched C₉-C₁₁ alkyl optionally substituted by one -OH;
- substituent of formula (II) 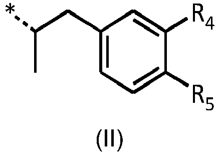 wherein either R₄ is H and R₅ is selected from the group consisting of methoxy and linear or branched C₃-C₆ alkyl; or R₄ and R₅ form together a -O-CH₂-O-group.

In another particular embodiment, in the compounds of formula (I), R₂ is linear or branched C₉-C₁₁ alkyl optionally substituted by one -OH.

Preferably, in the compounds of formula (I) R₂ is selected from the group consisting of: 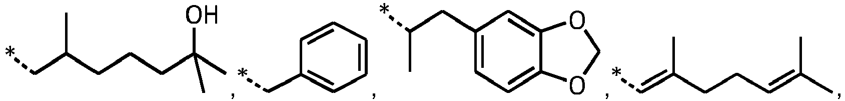 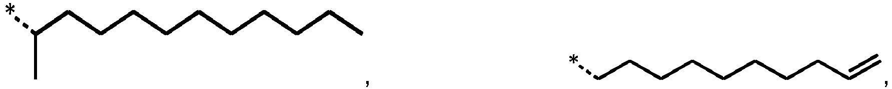 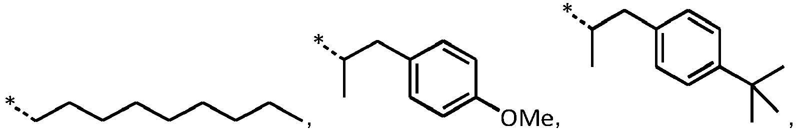 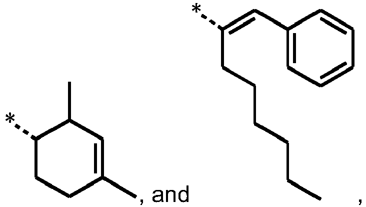 wherein the dotted line ending in * represents the attachment point to the rest of the molecule.

In a particular embodiment, in the substituent of formula (II), 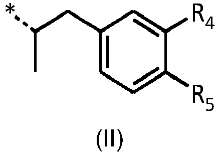 either R₄ is H and R₅ is selected from the group consisting of -O(C₁-C₃ alkyl) and linear or branched C₃-C₆ alkyl; or R₄ and R₅ form together a -O-CH₂-O- group. Preferably, R₄ is H and R₅ is selected from the group consisting of -O(C₁-C₃ alkyl) and linear or branched C₃-C₆ alkyl. More preferably, R₄ is H and R₅ is selected from the group consisting of methoxy and tert-butyl.

The dotted line ending in * representing the attachment point to the rest of the molecule represents the bond that is formed between R₂ and the carbon atom of the imine, i.e. in the -N=CH-R₂ moiety, the bond formed between the CH and the R₂ group. For example, when R₂ is 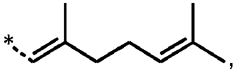 the compound of formula (I) would have the structure depicted below.

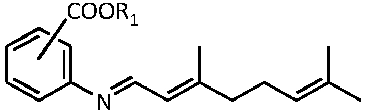

In a particular embodiment, in the compounds of formula (I) R₂ is selected from the group consisting of: 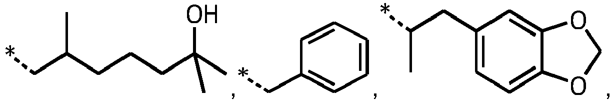 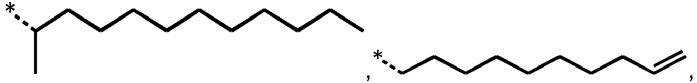 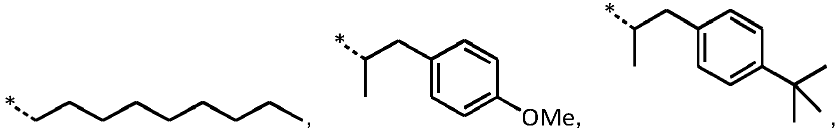 and 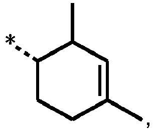 wherein the dotted line ending in * represents the attachment point to the rest of the molecule.

In another particular embodiment, in the compounds of formula (I) R₂ is selected from the group consisting of: 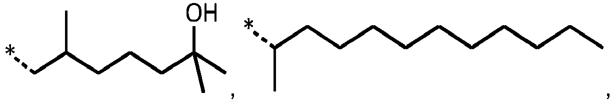 and 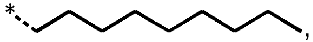 wherein the dotted line ending in * represents the attachment point to the rest of the molecule.

In another embodiment, the compound of formula (I) is selected from the group consisting of: 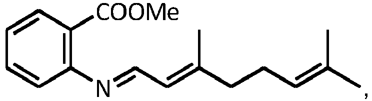 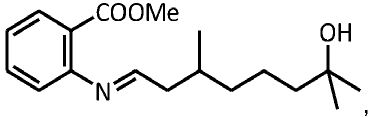 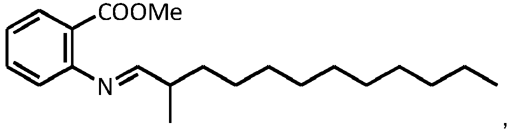 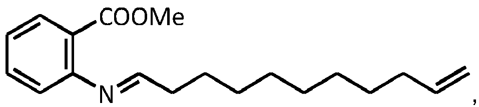 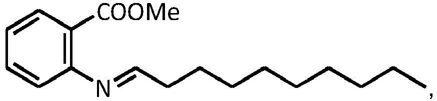 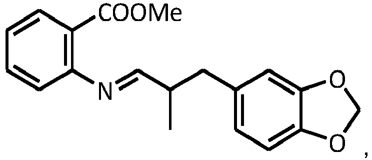 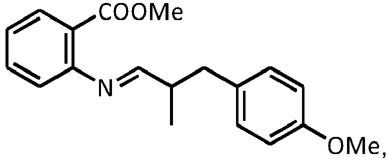 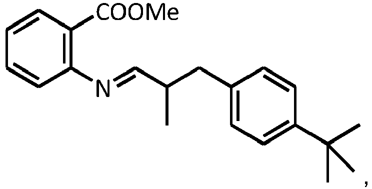 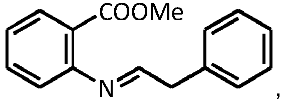 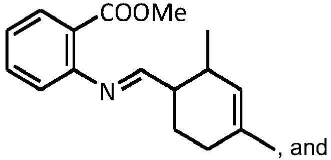 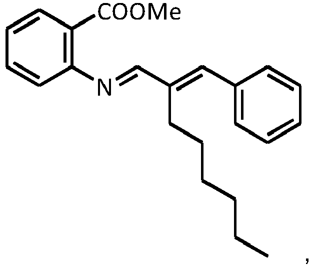 or a cosmetically acceptable salt, stereoisomer or metal complex thereof.

The compounds of formula (I) may be obtained by reaction of an amine of formula (III) with an aldehyde of formula (IV) 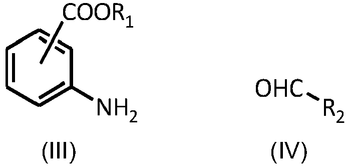 wherein R₁ and R₂ are as previously defined.

The formation of imines by reaction of an amine with an aldehyde is known in the art and has been described [Smith M.B. and March J., "March's Advanced Organic Chemistry: Reactions, Mechanisms, and Structure", 6th edition, Wiley-Interscience, John Wiley& Sons, Inc., New Jersey, 2007]. Amines (III) and aldehydes (IV) are commercially available, or their synthesis has been described in the literature. The reaction of amines (III) with aldehydes (IV) is usually carried out in the absence of a solvent or in the presence of an organic solvent, such as a glycol (i.e. dipropylene glycol, dipropylene glycol methyl ether, etc.), an alcohol (methanol, ethanol, 3-methoxy-3-methylbutan-1-ol), or toluene, preferably at a temperature of from 20 to 150 °C at atmospheric pressure or under reduced pressure. Since the reaction is reversible and water forms in the reaction medium, said water may be removed. Azeotropic distillation may be used, in particular when toluene is used as the solvent, preferably by means of a Dean-Stark apparatus. Alternatively, dehydration agents may be added to the reaction medium such as sodium sulfate and molecular sieves. Alternatively, dehydrating solvents may be used, such as tetramethyl orthosilicate or trimethyl orthoformate. The reaction can be accelerated by acid or base catalysis. In such cases, mineral acids, such as H₂SO₄ or HCl, organic acids, such as p-toluene sulfonic acid, pyridinium p-toluenesulfonate, acidic resin, montmorillonite, or Lewis acids (ZnCl₂, TiCl₄, SnCl₄, BF₃Et₂O, Mg(ClO₄)₂, MgSO₄), can be used. Bases such aminoalcohols (i.e. monoethanolamine, trietanolamine) or bases (i.e. CO₃²⁻, HCO₃⁻ can be also used. In particular, the molar ratio of amine (III) to aldehyde (IV) may be from 1:2 to 2:1, preferably about 1:1-1:1.5.

In the second aspect, the invention refers to the cosmetic use of a cosmetic composition comprising a compound of formula (I) or a cosmetically acceptable salt, stereoisomer or metal complex thereof as defined in the first aspect and a cosmetically acceptable excipient in the treatment and/or prevention of signs of skin aging, in particular of signs of skin photoaging caused by blue light. Preferably, the cosmetic use is in the prevention of signs of skin aging, in particular of signs of skin photoaging caused by blue light. Preferably, the signs of skin aging refer to wrinkles, hyperpigmentation, age spots and sagging of the skin.

Cosmetically acceptable excipients are well known in the art and refers to any substance that is present in the cosmetic compositions and which does not cause any adverse health reaction when applied to the skin.

Preferably, the cosmetically acceptable excipient is selected from the group consisting of solvents, surfactant, preservatives, rheology modifiers, antioxidants, chelating agents, UV filters, colorants, fragrance, fixative agents, and mixtures thereof.

In the context of the present invention, a "solvent" is a substance which is added to the cosmetic composition to dissolve other components such as the compounds of formula (I) and/or any cosmetically acceptable excipient. Examples of solvents suitable for the cosmetic compositions defined herein are water, ethanol, isopropanol, 3-methoxy-3-methyl-1-butanol; dipropylene glycol, (mono)propylene glycol, propanediol, ethyldiglycol, tripropylene glycol, diethyleneglycol monoethyl ether, butyldiglycol, pentylene glycol, triacetin, triethyl citrate, isopropyl myristate, diethyl phthalate, dioctyl adipate, paraffin, isoparaffin, and mixtures thereof.

The cosmetic composition may comprise from 0.01 to 99 wt% of a solvent with respect to the total weight of the cosmetic composition; preferably from 1 to 90 wt%; more preferably from 10 to 85 wt%; still more preferably from 20 to 80 wt%.

In the context of the present invention, a "surfactant" is a substance which decreases the surface tension of the composition with respect to the same composition in the absence of said substance and furthermore facilitates the uniform distribution of the composition when it is used (i.e. solubilizing effect). Examples of surfactants suitable for the cosmetic compositions defined herein are sodium laureth sulfate, sodium lauroyl sarcosinate, coco glucoside, sodium cocoyl sarcosinate, lauryl glucoside, (di)sodium cocoyl glutamate, cocamidopropyl betaine, hydrogenated castor oil (40-60 MOE); amine oxides such as mixtures of lauric/myristic amine oxide; PEG-20 sorbitan monolaurate, PEG-20 sorbitan monopalmitate, PEG-20 sorbitan, PEG-20 sorbitan tristearate, PEG-20 sorbitan monooleate, and mixtures thereof.

The cosmetic composition may comprise from 0.01 to 50 wt% of surfactants with respect to the total weight of the cosmetic composition; preferably from 1 to 30 wt%; more preferably from 1 to 25 wt%.

In the context of the present invention, a "preservative" refers to a substance which inhibits or reduces the development of micro-organisms in the cosmetic composition with respect to the same composition in the absence of said substance. Examples of preservatives suitable for the cosmetic compositions defined herein are benzoic acid, sorbic acid, lactic acid, phenoxyethanol, combinations of phenoxyelthanol with caprylyl glycol, ethylhexylglycerin, hydroxyacetophenone and/or decylene glycol, imidazolidinyl urea, diazolidinyl urea, ethylparaben, methylparaben, sodium methylparaben, propylparaben, sodium nitrite, sodium benzoate, sodium sorbate, methylisothiazolinone, methylchloroisothiazolinone, lactic acid, and combinations thereof.

The cosmetic composition may comprise from 0.01 to 10 wt% of preservatives with respect to the total weight of the cosmetic composition, preferably from 0.1 to 5 wt%; more preferably from 0.1 to 1 wt%.

In the context of the present invention, a "rheology modifier" refers to a substance which increases or decreases the viscosity of the composition with respect to the same composition in the absence of said substance. Examples of rheology modifiers suitable for the cosmetic compositions defined herein are xanthan gum, guar gum, carboxymethyl cellulose, carrageenan gum, ethyl cellulose, silicon dioxide, and mixtures thereof.

The cosmetic composition may comprise from 0.01 to 10 wt% of rheology modifiers with respect to the total weight of the cosmetic composition, preferably from 0.1 to 5 wt%; more preferably from 0.1 to 1 wt%.

In the context of the present invention, an "antioxidant" refers to a substance which inhibits or reduces reactions promoted by oxygen in the cosmetic composition with respect to the same composition in the absence of said substance, thus avoiding oxidation and rancidity. Examples of antioxidants suitable for the cosmetic compositions defined herein are butylated hydroxytoluene, butylated hydroxyanisole, tetradibutyl entaerythrityl hydroxyhydrocinnamate, acetyl zingerone, astaxanthin, trimethoxy benzylidene pentanedione, tocopherols (α, β-, γ-...), citric acid, L-ascorbic acid, and mixtures thereof.

The cosmetic composition may comprise from 0.01 to 10 wt% of antioxidants with respect to the total weight of the cosmetic composition, preferably from 0.1 to 5 wt%; more preferably from 0.1 to 2 wt%.

In the context of the present invention, a "chelating agent" refers to a substance which interacts to form complexes with metal ions which could affect stability and/or appearance of the cosmetic composition. Examples of chelating agents suitable for the cosmetic compositions defined herein are ethylenediaminetetraacetic acid, L-glutamic acid N,N-diacetic acid tetrasodium salt, L-aspartic acid, N,N'-1,2-ethanediylbis-, trisodium salt, mixtures of lauryl alcohol diphosphonic acid and lauric acid, bis-ethylhexyl hydroxydimethoxybenzylmalonate, and mixtures thereof.

The cosmetic composition may comprise from 0.01 to 5 wt% of chelating agents with respect to the total weight of the cosmetic composition, preferably from 0.1 to 2 wt%; more preferably from 0.1 to 1 wt%

in the context of the present invention a "UV filter" or "UV absorber" refers to a substance (or a mixture of substances) which are added to the cosmetic formulation in order to protect it from the deleterious effects of UV light. Examples of these substances include butyl methoxydibenzoylmethane, ethylhexyl methoxycinnamate, ethylhexyl salicylate, octocrylene, 3,3,5-trimethylcyclohexyl salicylate, 2-hydroxy-4-methoxybenzophenone, 4-methylbenzyliden camphor, titanium dioxide, zinc dioxide, butyl methoxydibenzoylmethane, ethylhexyl triazone, bis-ethylhexyloxyphenol methoxyphenyl triazin, diethylamino hydroxybenzoyl hexyl benzoate, diethylhexyl butamido triazone, terephthalylidene dicamphor sulfonic acid, drometrizol trisiloxane, and mixtures thereof.

The cosmetic composition may comprise from 0.1 to 20 wt% of UV filters with respect to the total weight of the cosmetic composition, preferably from 0.5 to 10 wt%; more preferably from 1 to 5 wt%

In the context of the present invention, a "colorant" is a substance which is added to the cosmetic composition to impart color to said composition. The colorants can be liposoluble, water soluble, or dispersible in water or organic solvents (such as glycol solvents). Examples of colorants are Cl 60730 (Ext Violet 2), Cl 19140 (Yellow 5), Cl 14700 (Red 4), fat orange (CI 2055), fat blue (CI 6554), red Violet (CI 62025), fat yellow (CI 47000), fat green (CI 6 1565), fat red (CI 261 05), and mixtures thereof.

The cosmetic composition may comprise from 0.00001 to 1 wt% of colorant with respect to the total weight of the cosmetic composition, preferably from 0.0001 to 0.1 wt%; more preferably from 0.001 to 1 wt%.

In the context of the present invention, a "fragrance", "fragrance material" or "fragrance ingredient" refers to ingredients which are added to a composition to contribute to the olfactive properties of the overall composition. Typically, a perfume material will be recognized as possessing odors in its own right and will be relatively volatile. Typical perfume materials are described and mentioned in "Perfume and Flavour Chemicals", Volumes I and II (Steffan Arctander (1969), "Flavor and Fragrance Materials", Allured Publishing Co. Wheaton, III. USA (in any of their yearly publications) Fragrance ingredients can be synthetic but also natural products. Examples of fragrance ingredients suitable for fragrancing cosmetic compositions are exemplified below. Within the synthetic ingredients, most functional groups include fragrance ingredients, as exemplified below:
- alcohols, such as citronellol, geraniol, linalool, dihydromyrcenol, nerol, terpineol, phenylethyl alcohol, catechol, eugenol, nor-patchoulenol, vanillin, ethyl vanillin, thymol, cis-3-hexenol, borneol;
- ethers such as diphenyl oxide, b-naphthyl ethyl ether methyl cedryl ether, galaxolide, ambroxan^{®} ([3aR-(3aα,5aβ,9aα,9bβ)]-dodecahydro-3a,6,6,9a-tetramethylnaphtho[2,1 -b]furan) and Anther^{®} (2-(3-methylbutoxy)ethylbenzene);
- aldehydes, such as (Z)-3,7-dimethylocta-2,6-dienal, α-methyl-1,3-benzodioxole-5-propionaldehyde, α-hexylcinnamaldehyde, hydroxycitronellal, decanal, 2,4-dimethylcyclohex-3-ene-1-carbaldehyde, 3-(p-methoxyphenyl)-2-methylpropionaldehyde, phenyl acetaldehyde, 2-methylundecanal, aldehyde C-11 undecylenic, butylphenyl methylpropional and anisaldehyde;
- ketones, such as α-ionone, camphor, isoraldein (isomethyl-α-ionone), methylionone, methyl cedryl ketone, 2-heptanone, tonalide, musk ketone, methyl naftyl ketone, and delta damascone;
- esters, such as terpenyl acetate, allyl phenoxyacetate, benzyl salicylate, cinnamyl propionate, citronellyl acetate, citronellyl ethoxolate amyl acetate, decyl acetate, dimethylbenzylcarbinyl acetate, dimethylbenzylcarbinyl butyrate, geranyl acetate, ethyl acetylacetate, hexenyl isobutyrate, linalyl acetate, methyl dihydrojasmonate, styrallyl acetate, vetiveryl acetate, and neryl acetate;
- lactones, such as 7-undecalactone,
- carboxylic acids such as hexanoic acid, phenylacetic acid, butyric acid, cinnamic acid, undecylenic acid, and acetic acid;
- linear, branched or aromatic unsubstituted structures also exhibit olfactory characteristics, terpenes being one representative family (which the isoprene as main characteristic structure). Terpene derivatives include δ-limonene and pinene.

Examples of natural ingredients used in fragrances include essential oils, absolutes, resins, resinoids, concretes balsams, exudates, extracts, such as tea tree oil, basil oil, citrus fruit oils (such as bergamot oil, orange oil, lemon oil, mandarin oil, etc.), rose absolute, myrtle oil, vetiver oil, patchouli oil, Petitgrain oil Paraguay, and Agaroud oil, among others.

The terms "perfume composition" or "fragrance composition" or plainly "fragrance" (all used synonymously in the present specification) are used herein to mean a mixture of fragrance materials, if desired mixed with or dissolved in a suitable solvent or mixed with a solid substrate. The mixture may be a complex mixture of many ingredients. The perfume composition is added to the cosmetic compositions to impart a pleasant smell and/or masking a bad smell.

The cosmetic composition may comprise from 0.01 to 50 wt% of fragrance with respect to the total weight of the cosmetic composition, preferably from 0.05 to 40 wt%; more preferably from 0.1 to 30 wt%.

In the context of the present invention, a "fixative agent" refers to a substance which modulates the volatility of fragrance components and help prolong the longevity of the aroma in the cosmetic composition with respect to the same composition in the absence of said substance. Examples of fixative agents compounds suitable for the cosmetic compositions defined herein are piperonylbutoxide, poly(PG)monobutylether, triethylene glycol, hercolyn D, glycerol triacetate, triethyl citrate, C9-11 linear alcohol ethoxylates, C12-15 linear alcohol ethoxylates, alcohols C11-15-secondary ethoxylated, PPG-7-buteth-10, PPG-4-ceteth-10, C14-15 linear alcohol ethoxylates alcohol ethoxylate, coconut fatty amine ethoxylate (10 mol EO), poly(ethylene glycol) methyl ether, PPG - 15 stearyl ether, polyoxyethylene (100) stearyl ether, tripropylene glycol mono n-butyl ether, octyl phenol ethoxylate, methyl hydrogenated rosinate, sucrose acetate isobutyrate, polycitronellol, sodium polystyrene sulfonate, acrylates/octylacrylamide copolymer, and mixtures thereof.

The cosmetic composition may comprise from 0.1 to 50 wt% of fixatives with respect to the total weight of the composition, preferably from 1 to 40 wt%; more preferably from 5 to 30 wt%.

The compounds of formula (I) present in the cosmetic compositions used in the second aspect are as defined for the first aspect.

Preferably, the cosmetic composition comprises from 0.01 to 30 wt% of the compound of formula (I) or a cosmetically acceptable salt, stereoisomer or metal complex thereof, with respect to the total weight of the cosmetic composition; more preferably from 0.05 to 20 wt%; even more preferably from 0.1 to 10 wt%; still more preferably from 0.5 to 5 wt%.

The cosmetic compositions defined herein are prepared using conventional means in the art, such as mixing the components of the composition. Examples of cosmetically acceptable excipients and their amounts have been described above. The appropriate cosmetically acceptable excipient and their amounts in a specific composition can readily be determined by those skilled in the art according to the type of composition being prepared.

The cosmetic compositions can be provided in any form suitable for applying the compositions to the skin, preferably as a solid, a powder, a lotion, a cream, a gel, a paste, a serum, a mask, an oil, a foam, a mousse, a stick or an aerosol.

Preferably, the cosmetic composition is topically applied to the skin, preferably one, two or three times a day.

As previously explained, in the absence of water removal, the formation of the compounds of formula (I) does not usually evolve to completion and in equilibrium usually part of the precursors still available. Thus, in the presence of the individual counterparts amine (III) and aldehyde (IV) as defined above, evolution of the overall mixture can lead to partial formation of the compound of formula (I) over the time. Thus, when amines (III) and aldehydes (IV) as previously defined are comprised in the same composition, they may lead to some extent to the formation of compounds of formula (I). Thus, the use of these compositions comprising amines (III) and aldehydes (IV) as previously defined are also included in the scope of this invention.

The above aspect may also be formulated as a compound of formula (I) or a cosmetically acceptable salt, stereoisomer or metal complex thereof as defined in the first aspect, or of a cosmetic composition as defined in the second aspect for use in the treatment and/or prevention of signs of skin aging, in particular signs of skin photoaging caused by blue light; preferably, for the prevention of signs of skin aging, in particular of signs of skin photoaging caused by blue light. Preferably, the signs of skin aging refer to wrinkles, hyperpigmentation, age spots and sagging of the skin.

A further aspect refers to a method for the cosmetic (non-therapeutic) treatment and/or prevention of signs of skin aging, in particular signs of skin photoaging caused by blue light, comprising the topical application of a compound of formula (I) or a cosmetically acceptable salt, stereoisomer or metal complex thereof as defined in the first aspect, or of a cosmetic composition as defined in the second aspect. Preferably, the method is in the prevention of signs of skin aging, in particular of signs of skin photoaging caused by blue light. Preferably, the signs of skin aging refer to wrinkles, hyperpigmentation, age spots and sagging of the skin.

Preferably, the topical application is performed one, two or three times a day.

The following examples represent specific embodiments of the present invention. They do not intend to limit in any way the scope of the invention defined in the present description.

### Examples

### Synthesis of the example compounds

The synthesis of the different Schiff bases was performed by adapting the methods described in the art (for instance in EP0480000B1 to Givaudan). Briefly, into a reaction flask equipped with a stirrer and a thermometer, equal amounts by weight of aldehyde of formula (IV) and methyl anthranilate are added. The reactor is heated slowly to 90 °C, then maintained at that temperature until the reaction does not progress anymore (typically between 2 and 8 hours). The reaction is monitored by gas chromatography (GC). The reaction mass may be used as it is without further purification. Confirmation by GC analysis.

GC conditions: (Agilent Technologies); Non-polar capillary column 5 % Phenyl Dimethyl Polysiloxan (Length = 30 m; Internal diameter = 0.25 mm; Film thickness = 0.25 µm); Helium as carrier; programmed as follows: 3.5 min at 55 °C, followed by i) 5.5 °C/min slope until 100 °C (hold for 3 min); ii) 8 °C/min slope until 200 °C (hold for 15 min) and iii) 8 °C/min slope until 240 °C (hold for 20 min). 1 µL injection using Split (90:1 ratio).

The following products have been prepared according to this method:
i) 3-(p-methoxyphenyl)-2-methylpropionaldehyde / methyl anthranilate Schiff Base (SB 1). The reaction mass is used crude without any purification. The yellow viscous liquid mixture contains 1.38% of aldehyde, 10.6% of methyl anthranilate, and 79.1% of the Schiff Base 1 (by weight of the total weight of the mixture, % w/w). GC retention times for the Schiff Base are 58.9 min and 63.1 min (corresponding to the d- and I- isomers); m/z=77.1, 121.1, 150.0, 161.1, 202.0, 296.1.
ii) 2,4-dimethylcyclohex-3-ene-1-carbaldehyde / methyl anthranilate Schiff Base (SB 2). The reaction mass is used crude without any purification. The yellow viscous liquid mixture contains 1.47% of aldehyde, 11.4% of methyl anthranilate, and 80.5% of the Schiff Base 2 (by weight of the total weight of the mixture, % w/w). GC retention times for the Schiff Base are 46.0 min and 46.7 min (corresponding to the two stereoisomers formed); m/z=121, 191.0, 196.0, 256.0, 271.0.
iii) Phenyl acetaldehyde / methyl anthranilate Schiff Base (SB 3). The reaction mass is used crude without any purification. The yellow viscous liquid mixture contains 8.5% of aldehyde, 32.5% of methyl anthranilate, and 36.5% of the Schiff Base 3 (by weight of the total weight of the mixture, % w/w). GC retention times for the Schiff Base are 46.6 min and 50.0 min; (corresponding to the two stereoisomers formed) m/z=91.1, 165.1, 193.1, 220.1, 253.1.
iv) α-hexylcinnamaldehyde / methyl anthranilate Schiff Base (SB 4). The reaction mass is used crude without any purification. The yellow viscous liquid mixture contains 50.0% of aldehyde, 33.3% of methyl anthranilate, and 15% of the Schiff Base (by weight of the total weight of the mixture, % w/w). GC retention times for the Schiff Base are 63.5 min and 67.8 min (corresponding to the two stereoisomers formed); m/z=77.1, 91.1, 115.1, 214, 232, 246, 264, 292.
v) 2-methylundecanal / methyl anthranilate Schiff Base (SB 5). The reaction mass is used crude without any purification. The yellow viscous liquid mixture contains 0.8% of aldehyde, 8.6% of methyl anthranilate, and 84% of the Schiff Base (by weight of the total weight of the mixture, % w/w). GC retention times for the Schiff Base are 51.5 min and 55.9 min (corresponding to the two stereoisomers formed); m/z=132.0, 144.1, 172.1, 204.1, 260.1.
vi) α-methyl-1,3-benzodioxole-5-propionaldehyde / methyl anthranilate Schiff Base (SB 6). The reaction mass is used crude without any purification. The yellow viscous liquid mixture contains 13.5% of aldehyde, 19.9% of methyl anthranilate, and 16% of the Schiff Base 6 (by weight of the total weight of the mixture, % w/w). GC retention times for the Schiff Base are 65.0 min and 70.9 min (corresponding to the two stereoisomers formed). m/z= 77.1, 135.1, 145.6, 175.1, 278.1.
vii) Hydroxycitronellal / methyl anthranilate Schiff Base (SB 7). The reaction mass is used crude without any purification. The yellow viscous liquid mixture contains 15% of aldehyde, 17% of methyl anthranilate, and 68% of the Schiff Base 7 (by weight of the total weight of the mixture, % w/w). Confirmation by GC analysis. GC retention times for the Schiff Base are 47.5 min and 51.0 min (corresponding to the two stereoisomers formed) m/z= 43, 58, 59, 69, 81, 95, 109, 172, 204, 275
viii) (Z)-3,7-dimethylocta-2,6-diena / methyl anthranilate Schiff Base (SB 8). The reaction mass is used crude without any purification. The yellow viscous liquid mixture contains 5.3% of aldehyde, 27.8% of methyl anthranilate, and 50.8% of the Schiff Base 8 (by weight of the total weight of the mixture, % w/w). GC retention times for the Schiff Base are 50.8 min and 53.8 min (corresponding to the two stereoisomers formed); m/z=173, 205, 285.
ix) Aldehyde C-11 (i.e. 10-undecenal) methyl anthranilate Schiff Base (SB 9). The reaction mass is used crude without any purification. The yellow viscous liquid mixture contains 7.9% of aldehyde, 26.8% of methyl anthranilate, and 12.5% of the Schiff Base 9 (by weight of the total weight of the mixture, % w/w). GC retention times for the Schiff Base are 50.1 min and 53.2 min (corresponding to the two stereoisomers formed); m/z=158.1, 177.3, 190.1, 301.1.
x) Butylphenyl methylpropional / methyl anthranilate Schiff Base (SB 10). The reaction mass is used crude without any purification. The yellow viscous liquid mixture contains 0.5% of aldehyde, 14.3% of methyl anthranilate, and 80% of the Schiff Base10 (by weight of the total weight of the composition, % w/w). GC retention times for the Schiff Base are 61.5 min and 66.7 min (corresponding to the two stereoisomers formed); m/z=57.0, 131.0, 147.0, 290.0, 246.0.

### UV-Visible absorption and photostability

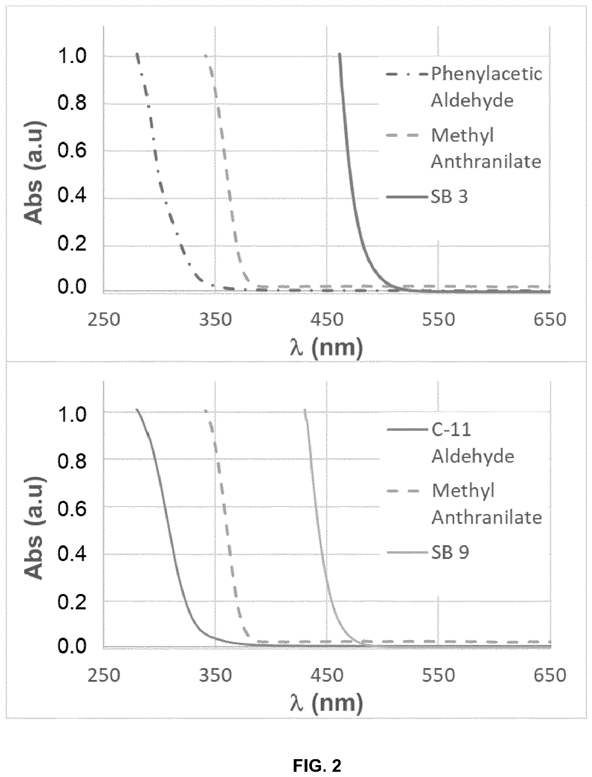

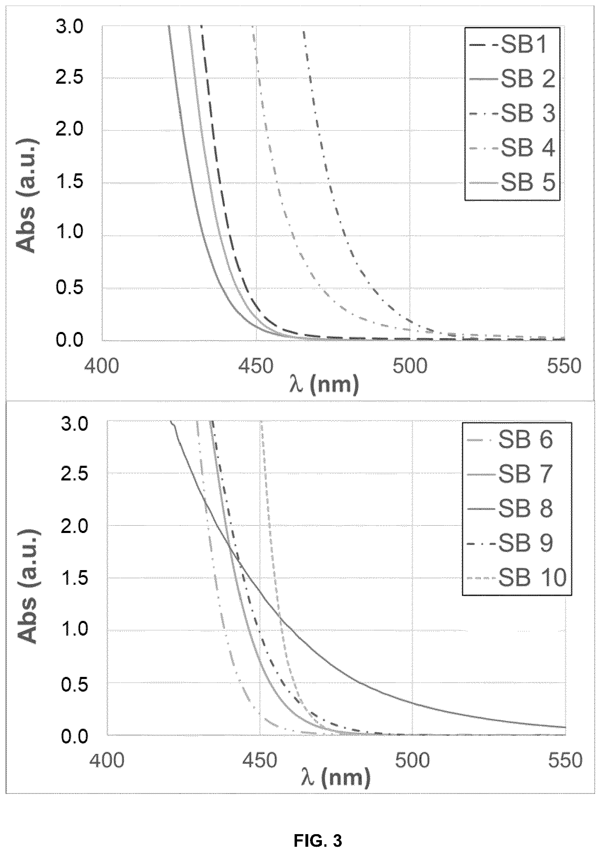

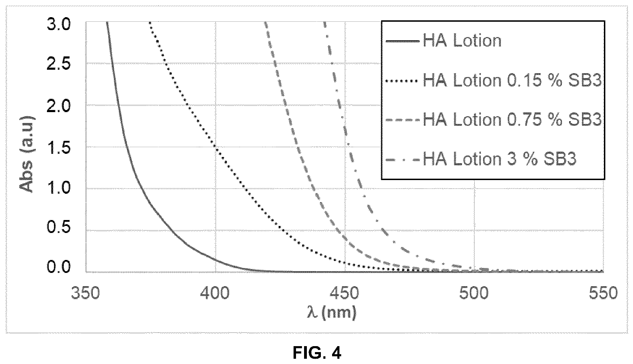

UV-Vis absorption spectra were recorded in a double-beam spectrophotometer using 1 cm quartz cuvettes. Figure 3 evidence the absorption spectra of Schiff Bases SB1 to SB 10 described above, showing their blue light absorption capacity. Figure 4 also ratifies the blue-light absorption capacity of a fragranced composition including a Schiff Base, namely SB 3, at different concentrations (0.15 wt%, 0.75 wt% and 3 wt% of SB 3 with respect to the total weight of the composition), in a hydroalcoholic splash (composed of 90 wt% ethanol, 2 wt% 3-methoxy-3-methyl-1-butanol, 6 wt% water and 2 wt% fragrance EMSA Citrus described in the examples below, all in parts by weight with respect to the total weight of the hydroalcoholic splash). The results on Figure 2 show that the precursors used in the synthesis of the Schiff bases do not absorb in the blue region of the UV-Vis spectrum whereas the resulting Schiff Bases do absorb within this region, evidencing their ageing protecting effects against blue light.

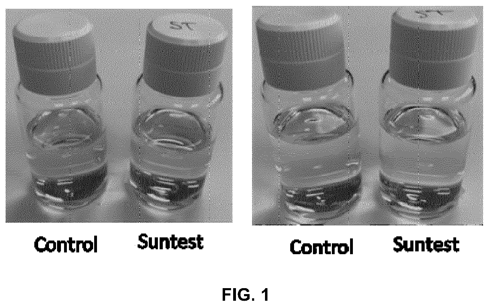

Photostability tests were performed using a photostability chamber (ATLAS-Xenotest SUNTEST CPS+). Samples (i) Phenyl acetaldehyde / methyl anthranilate Schiff base; and ii) Hydroxycitronellal / methyl anthranilate Schiff base) were exposed to light cycles (3x8 h with a 500 W/m² irradiance; Xenon lamp plus Suprax UV and Window Glass filters). As shown in Figure 1, the compounds of the invention remain stable. Only a reduction of concentration below 10% was observed as compared to the dark control at the end of the photostability test, which anticipates its functionality over its lifetime.

### Example cosmetic compositions

Citrus Type Fragrance- "EMSA Citrus" used in the example cosmetic compositions described below has the following ingredients:

| **Ingredient** | **Parts by weight (g)** |
|---|---|
| Berqamote oil | 250 |
| Dipropyleneglycol | 100 |
| Orange Oil | 150 |
| Lemon Oil | 75 |
| Pyranol | 60 |
| Linalool | 50 |
| Petitgrain Paraguay Oil | 50 |
| Terpenyl Acetate | 40 |
| Tonalide | 40 |
| Galaxolide | 30 |
| Hedione | 30 |
| Hydroxycitronellal | 30 |
| Phenylethyl alcohol | 20 |
| Musk ketone | 20 |
| D-limonene | 20 |
| b-naphthyl ethyl ether | 15 |
| Methyl naphthyl ketone | 10 |
| Damascone delta | 10 |
| **Total (g)** | **1000** |

Example compositions 1-5 have been prepared.

### Example composition 1. Eau de toilette

| **Ingredient** | **Parts by weight (%)** |
|---|---|
| Ethanol | 80 |
| Fragrance EMSA Citrus | 8 |
| Phenylacetic acetaldehyde / methyl anthranilate Schiff base (SB3) | 2 |
| Antioxidants, colorants | *q.s.* |
| Water | ad. 100 |

### Example composition 2. Hydroalcoholic lotion splash

| **Ingredient** | **Parts by weight (%)** |
|---|---|
| Ethanol | 78 |
| Propylene Glycol | 2 |
| Fragrance EMSA Citrus | 4 |
| Phenylacetic acetaldehyde / methyl anthranilate Schiff base (SB3) | 1 |
| Preservatives, antioxidants, | *q.s.* |
| Water | ad. 100 |

### Example composition 3. Face, neck and body Oil

| **Ingredient** | **Parts by weight (%)** |
|---|---|
| Paraffin Oil | 10 |
| Almond Oil | 5 |
| Isopropyl Myristate | 5 |
| Fragrance EMSA Citrus | 0.7 |
| Phenylacetic acetaldehyde / methyl anthranilate Schiff base (SB3) | 0.3 |
| Cetyl Palmitate | 3 |
| Antioxidants | *q.s.* |
| Caprylic/capric Triglycerides | *ad. 100* |

### Example composition 4. Clear perfumed skin gel

| **Ingredient** | **Parts by weight (%)** |
|---|---|
| PEG-40 hydrogenated castor oil | 8 |
| Propylene Glycol | 1 |
| Fragrance EMSA Citrus | 3 |
| Phenylacetic acetaldehyde / methyl anthranilate Schiff base (SB3) | 1 |
| Carbopol | 3.5 |
| Preservatives, antioxidants | *q.s.* |
| Water | ad. 100 |

### Example composition 5. All-purpose solid perfume

| **Ingredient** | **Parts by weight (%)** |
|---|---|
| Caprylic capric triglycerides | 35 |
| Cetylic alcohol | 35 |
| Karite butter | 10 |
| Vegetable wax | 10 |
| Coco oil | 10 |
| Fragrance EMSA Citrus | 6 |
| Phenylacetic acetaldehyde / methyl anthranilate Schiff base (SB3) | 1 |
| Preservatives, antioxidants | *q.s.* |
| Caprylic capric triglycerides | ad. 100 |

The example compositions described above may be prepared by mixing the ingredients at the concentrations described in the table at room temperature under gentle stirring.

In the skin gel, carbomer is pre-dispersed in water at 2% (getting a translucent solution) and the following ingredients are added in listed order.

The solid perfume may be prepared by blending all ingredients at 100 °C and adding the fragrance at around 75-80 °C.

## Claims

**1.** A cosmetic use of a compound of formula (I) 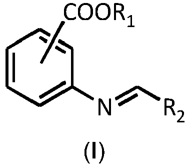 wherein
R₁ is selected from the group consisting of linear or branched C₁-C₆ alkyl, linear or branched C₂-C₆ alkenyl and hydrogen atom;
R₂ is selected from the group consisting of:
• linear or branched C₁-C₁₂ alkyl optionally substituted by one or more substituents selected from the group consisting of -OH; phenyl which is in turn optionally substituted by one or more substituents selected from -OH, -OR₃ and R₃; and benzodioxolyl;
• linear or branched C₂-C₁₂ alkenyl optionally substituted by one or more substituents selected from the group consisting of -OH and phenyl;
• C₅-C₆ cycloalkenyl optionally substituted by one or more substituents selected from the group consisting of linear or branched C₁-C₆ alkyl and linear or branched C₂-C₆ alkenyl, and wherein said alkyl and alkenyl are in turn optionally substituted by a -OH;
• C₅-C₆ cycloakyl optionally substituted by one or more substituents selected from the group consisting of linear or branched C₁-C₆ alkyl and linear or branched C₂-C₆ alkenyl, and wherein said alkyl and alkenyl are in turn optionally substituted by a -OH;
• phenyl optionally substituted by -OH and -OR₆; and
R₃ is independently a linear or branched C₁-C₆ alkyl; and
R₆ is a linear or branched C₁-C₃ alkyl
or a cosmetically acceptable salt, stereoisomer or metal complex thereof, in the treatment and/or prevention of signs of skin aging.

**2.** The cosmetic use according to claim 1, wherein the -COOR₁ group is in ortho position with respect to the -N=CH-R₂ moiety.

**3.** The cosmetic use according to any one of the preceding claims, wherein R₁ is selected from the group consisting of linear or branched C₁-C₆ alkyl and linear or branched C₂-C₆ alkenyl; preferably R₁ is C₁-C₃ alkyl.

**4.** The cosmetic use according to any one of the preceding claims, wherein R₁ is methyl.

**5.** The cosmetic use according to any one of the preceding claims, wherein R₂ is selected from the group consisting of:
• linear or branched C₇-C₁₁ alkyl optionally substituted by one -OH;
• linear or branched C₇-C₁₁ alkenyl optionally substituted by one phenyl;
• cyclohexenyl optionally substituted by one or two substituents selected from the group consisting of linear or branched C₁-C₆ alkyl and linear or branched C₂-C₆ alkenyl;
• cyclohexyl optionally substituted by one substituent selected from the group consisting of linear or branched C₁-C₆ alkyl which is in turn optionally substituted by a -OH;
• phenyl optionally substituted by methoxy;
• benzyl;
• phenylethenyl; and
• a substituent of formula (II): 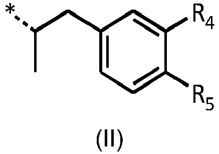 wherein
the dotted line ending in * represents the attachment point to the rest of the molecule; and
either R₄ is H and R₅ is selected from the group consisting of -OR₃, R₃ and OH, wherein R₃ is independently a linear or branched C₁-C₆ alkyl;
or R₄ and R₅ form together a -O-CH₂-O- group.

**5.** The cosmetic use according to any one of the preceding claims, wherein R₂ is selected from the group consisting of:
• linear or branched C₉-C₁₁ alkyl optionally substituted by one -OH;
• linear or branched C₈-C₁₀ alkenyl optionally substituted by one phenyl;
• cyclohexenyl optionally substituted by one or two substituents selected from the group consisting of linear or branched C₁-C₃ alkyl;
• phenyl optionally substituted by methoxy; and
• a substituent of formula (II) wherein either R₄ is H and R₅ is selected from the group consisting of methoxy and linear or branched C₃-C₆ alkyl; or R₄ and R₅ form together a -O-CH₂-O- group.

**6.** The cosmetic use according to any one of the preceding claims, wherein R₂ is selected from the group consisting of: 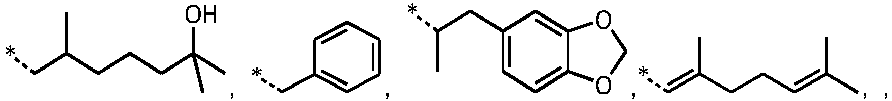 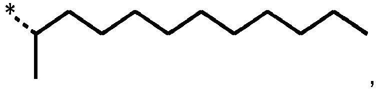 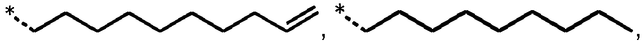 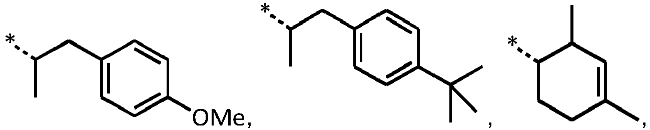 and 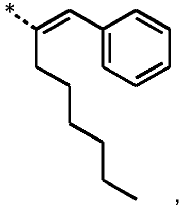 wherein the dotted line ending in * represents the attachment point to the rest of the molecule.

**7.** A cosmetic use of a cosmetic composition comprising a compound of formula (I) or a cosmetically acceptable salt, stereoisomer or metal complex thereof as defined in any one of the preceding claims and a cosmetically acceptable excipient in the treatment and/or prevention of signs of skin aging.

**8.** The cosmetic use according to claim 7, wherein the cosmetically acceptable excipient is selected from the group consisting of solvents, surfactant, preservatives, rheology modifiers, antioxidants, chelating agents, UV filters, colorants, fragrance, fixative agents, and mixtures thereof.

**9.** The cosmetic use according to claim 7 or 8, wherein the composition comprises from 0.01 to 20 wt% of the compound of formula (I) or a cosmetically acceptable salt, stereoisomer or metal complex thereof, with respect to the total weight of the composition.

**10.** The cosmetic use according to any one of claims 7 to 9 wherein the cosmetic composition is in the form of a solid, a powder, a lotion, a cream, a gel, a paste, an oil, a serum, a mask, a foam, a mousse, a stick or an aerosol.

**11.** The cosmetic use according to any one of the preceding claims, wherein the signs of skin aging are selected from the group consisting of wrinkles, hyperpigmentation, age spots and sagging of the skin.

**12.** The cosmetic use according to any one of the preceding claims, wherein the compound of formula (I) or the cosmetically acceptable salt, stereoisomer or metal complex thereof as defined in any one of claims 1 to 6, or the cosmetic composition as defined in any one of claims 7 to 10, is topically applied to the skin, preferably one, two or three times a day.

**13.** A method for the cosmetic treatment and/or prevention of signs of skin aging, comprising the topical application of a compound of formula (I) or a cosmetically acceptable salt, stereoisomer or metal complex thereof as defined in any one of claims 1 to 6, or of a cosmetic composition as defined in any one of claims 7 to 10.

**14.** The method according to claim 13, wherein the topical application is performed one, two or three times a day.

**15.** The method according to claim 13 or 14 wherein the signs of skin aging are selected from the group consisting of wrinkles, hyperpigmentation, age spots and sagging of the skin.
